(19) **Europäisches Patentamt / European Patent Office / Office européen des brevets**

(11) **EP 1 591 110 B1**

(12) **EUROPEAN PATENT SPECIFICATION**

(45) Date of publication and mention
of the grant of the patent:
**18.03.2015 Bulletin 2015/12**

(21) Application number: **04704362.5**

(22) Date of filing: **22.01.2004**

(51) Int Cl.:
*A61K 9/70* (2006.01)  *A61P 27/02* (2006.01)

(86) International application number:
**PCT/JP2004/000520**

(87) International publication number:
**WO 2004/064817 (05.08.2004 Gazette 2004/32)**

(54) **PERCUTANEOUS ABSORPTION PREPARATION FOR TREATING OPHTHALMIC DISEASE, USE THEREOF AND METHOD FOR MIGRATION OF OPHTHALMIC REMEDY INTO TOPICAL TISSUE IN EYE**

PERKUTANES ABSORPTIONSPRÄPARAT ZUR BEHANDLUNG VON AUGENERKRANKUNGEN, SEINE VERWENDUNG UND VERFAHREN ZUR MIGRATION DES AUGENPRÄPARATS IN DAS TOPISCHE GEWEBE EINES AUGES

PREPARATION A ABSORPTION PERCUTANEE POUR TRAITER UNE MALADIE OPHTALMIQUE, UTILISATION DE CETTE PREPARATION ET PROCEDE POUR FAIRE MIGRER UN MEDICAMENT OPHTALMIQUE DANS UN TISSU TOPIQUE DE L'OEIL

(84) Designated Contracting States:
**AT BE BG CH CY CZ DE DK EE ES FI FR GB GR HU IE IT LI LU MC NL PT RO SE SI SK TR**

(30) Priority: **22.01.2003 JP 2003013339**

(43) Date of publication of application:
**02.11.2005 Bulletin 2005/44**

(73) Proprietor: **Senju Pharmaceutical Co., Ltd.**
**Osaka-shi, Osaka 541-0046 (JP)**

(72) Inventors:
• **KAWAHARA, Kohji;**
**c/o NICHIBAN CO., LTD.**
**Kokyo, 1128663 (JP)**
• **ARAMOMI, Yasuhiko;**
**c/o NICHIBAN CO., LTD.**
**Kyo-ku, Tokyo, 1128663 (JP)**
• **OHTORI, Akira;**
**Osaka 541-0046 (JP)**

• **ISOWAKI, Akiharu**
**Osaka 541-0046 (JP)**

(74) Representative: **Jones, Helen M.M.**
**Gill Jennings & Every LLP**
**The Broadgate Tower**
**20 Primrose Street**
**London EC2A 2ES (GB)**

(56) References cited:
EP-A- 1 221 315     WO-A-02/22109
WO-A-99/51212      WO-A1-01/17527
WO-A1-01/26648     WO-A1-92/03133
WO-A1-97/24112     US-A- 4 052 505
US-A- 4 069 307     US-A- 4 144 317
US-A- 4 931 279

• DATABASE WPI Week 200007 Derwent Publications Ltd., London, GB; AN 2000-086657 XP002440604 & WO 99/60948 A2 (GRISHIN E V) 2 December 1999 (1999-12-02)

**Description**

TECHNICAL FIELD

The present invention relates to a novel transdermal drug delivery system for treatment of ophthalmic diseases, and particularly to a transdermal drug delivery system for treatment of ophthalmic diseases having a structure that a plaster layer containing a remedy for ophthalmic diseases is provided on a support. The present invention also relates to use of the transdermal drug delivery system for treatment of ophthalmic diseases. The present invention further relates to a method for percutaneously transferring a remedy for ophthalmic diseases to an ophthalmic topical tissue using the transdermal drug delivery system for treatment of ophthalmic diseases.

The transdermal drug delivery system for treatment of ophthalmic diseases according to the present invention is such that it can be applied to a skin surface including a front surface of an eyelid to percutaneously transfer the remedy for ophthalmic diseases in the plaster layer to an ophthalmic topical tissue, does not cause such side effects as observed in systemic remedies through a systemic blood flow and can sustainedly develop its efficacy.

BACKGROUND ART

As preparations for treatment of ophthalmic diseases, are known, for example, an ophthalmic solution, an ophthalmic ointment and an oral preparation. The ophthalmic solution containing a remedy for ophthalmic diseases is excellent in quick effectiveness, but is liable to be washed out by tears and poor in the sustainability of its efficacy. A preservative is generally added to the ophthalmic solution for preservation. However, this preservative tends to form the cause of stimulus. The ophthalmic ointment is better in the sustainability of drug efficacy than the ophthalmic solution, but it is difficult to exactly control the dose of the remedy for ophthalmic diseases in this ointment. In addition, the ophthalmic ointment may cause reduction in visual acuity upon its application in some cases. The oral preparation is excellent in the sustainability of its efficacy, but tends to cause side effects at other parts than the diseased part by systemic effect.

The treatment for an ophthalmic disease requires a system for treatment of ophthalmic diseases, by which a remedy for ophthalmic diseases can be sustainedly administered (topically administered) in an amount effective for treatment to an ophthalmic topical tissue such as the conjunctiva or cornea for enhancing therapeutic effect, and there is no fear of causing side effects. For example, in treatment for allergic conjunctivitis and infectious diseases, and prevention of a complication after a surgical operation for cataract, it is desired to sustain the efficacy of a remedy used over a relatively long period of time. However, there has heretofore been proposed no preparation for treatment of ophthalmic diseases that can sufficiently meet such a requirement.

On the other hand, transdermal drug delivery systems of a structure that a pressure-sensitive adhesive layer containing a drug is provided on a support, such as anti-inflammatory-analgesic patches, have been known (for example, Japanese Patent Application Laid-Open No. 2000-256214). Such a transdermal drug delivery system is generally either a systemic preparation for systemically administering a drug or that used by applying it to a skin surface of an elbow, knee, waist, shoulder or the like.

Transdermal drug delivery systems have heretofore been proposed in the technical field of preparations for treatment of ophthalmic diseases, also. For example, a percutaneously therapeutic system having a support layer, pressure-sensitive adhesive storage layer and a releasable protective layer, in which the storage layer comprises a polymer material and a pilocarpine base or a salt thereof, has been proposed (Japanese Patent Application Laid-Open No. 8-509716 through PCT route). In this percutaneously therapeutic system, the drug is not topically applied, but pilocarpine that is an active substance is systemically released from a skin surface to reduce an intraocular pressure.

An ophthalmic percutaneous-absorption patch comprising a drug-containing layer obtained by containing a drug to be delivered to any part of the posterior segment of the eye including the lens, the vitreous body, the choroid and the retina, and a percutaneous absorption enhancer in a base matrix has also be proposed (WO 01/26648). This ophthalmic percutaneous-absorption patch is used for delivering the drug to a diseased topical part of the posterior segment of the eye and is a kind of systemic preparation.

There has been proposed a therapy that a remedy for a dry eye disease is administered in the preparation form of a percutaneous patch or pad, in addition to a therapy that the remedy is topically administered in the form of an ophthalmic solution or ophthalmic ointment (U.S. Patent No. 6,277,855). This percutaneous patch or pad is used for bringing the remedy for the dry eye disease into contact with a lacrimal tissue by systemically absorbing and circulating the remedy and is a kind of systemic preparation.

When the transdermal drug delivery system is a systemic preparation, the drug permeates the surface of a skin patched and is absorbed in an intraepithelial blood capillary, and its efficacy is developed through a systemic blood flow from the blood capillary. In other words, the drug is systemically released through the systemic blood flow in a percutaneous manner, and a part thereof is delivered to a diseased part. Such a transdermal drug delivery system for systemic administration is not always effective for treatment of an ophthalmic disease that is a disease of an ophthalmic topical tissue as described below.

First, since the conventional transdermal drug delivery systems for systemic administration systemically release the remedy for ophthalmic diseases through the systemic blood flow in the percutaneous manner, it takes a long time to deliver the remedy to the ophthalmic topical tissue, and it is difficult to deliver the remedy in an amount effective for treatment to the ophthalmic topical tissue.

Second, when the conventional transdermal drug delivery systems for systemic administration systemically release a great amount of the remedy for the purpose of delivering the remedy in an amount effective for treatment to the ophthalmic topical tissue, there is a strong possibility that side effects may occur at other parts than the diseased part.

Third, the conventional transdermal drug delivery systems for systemic administration are difficult to selectively deliver the remedy in an amount effective for treatment to, for example, an external ophthalmic tissue such as the conjunctiva, lacrimal tissue or cornea, located on a rear surface of the eyelid. In other words, the transdermal drug delivery systems for systemic administration are not suitable for being selectively administered to the external ophthalmic tissue like the ophthalmic solution to develop the efficacy.

On the other hand, when the transdermal drug delivery system is a topical preparation, it is generally used by applying it to a skin surface of a diseased part of an elbow, knee, waist, shoulder or the like of the human body for the purpose of anti-inflammation, analgesia, etc. and is not intended to treat ophthalmic diseases.

DISCLOSURE OF THE INVENTION

It is an object of the present invention to provide a novel preparation for treatment of ophthalmic diseases, by which a remedy for ophthalmic diseases can be sustainedly administered in an amount effective for treatment to an ophthalmic topical tissue and its efficacy can be sustainedly developed without causing side effects.

More specifically, the object of the present invention is to provide a transdermal drug delivery system for treatment of ophthalmic diseases, by which a remedy for ophthalmic diseases can be percutaneously transferred in an amount effective for treatment to an external ophthalmic tissue such as the conjunctiva, lacrimal tissue or cornea, located on a rear surface of an eyelid in a relatively short period of time, and the efficacy thereof can be sustainedly developed.

Another object of the present invention is to provide use of the transdermal drug delivery system for treatment of ophthalmic diseases for applying the transdermal drug delivery system for treatment of ophthalmic diseases to a skin surface including a front surface of an eyelid to transfer a remedy for ophthalmic diseases in the plaster layer to an external ophthalmic tissue by percutaneous permeation substantially without being administered through a systemic blood flow.

A further object of the present invention is to provide a method for transferring a remedy for ophthalmic diseases to an external ophthalmic tissue by applying the transdermal drug delivery system for treatment of ophthalmic diseases to a skin surface including a front surface of an eyelid to transfer the remedy for ophthalmic diseases in a plaster layer by percutaneous permeation to the external ophthalmic tissue substantially without being administered through a systemic blood flow.

The present inventors have carried out an extensive investigation with a view toward achieving the above objects. As a result, the inventors have conceived of a transdermal drug delivery system for treatment of ophthalmic diseases having a structure that a plaster layer containing a remedy for ophthalmic diseases and a pressure-sensitive adhesive is provided on a support and equipped with a form that can be applied to a skin surface including a front surface of an eyelid. The invention is as defined in claim 1.

The transdermal drug delivery system for treatment of ophthalmic diseases according to the present invention is applied to the skin surface including the front surface of the eyelid, whereby the remedy for ophthalmic diseases in the plaster layer can be administered by percutaneous permeation to the external ophthalmic tissue substantially without being administered through a systemic blood flow. More specifically, when the transdermal drug delivery system for treatment of ophthalmic diseases according to the present invention is applied to a skin surface, the remedy for ophthalmic diseases permeates the skin patched to reach an external ophthalmic tissue such as the conjunctiva, lacrimal tissue or cornea, and the efficacy thereof can be developed. The pressure-sensitive adhesive is a rubber-based pressure-sensitive adhesive, acrylic pressure-sensitive adhesive or silicone-based pressure-sensitive adhesive.

In the present invention, the fact that the remedy for ophthalmic diseases in the plaster layer is administered by percutaneous permeation to the external ophthalmic tissue substantially without being administered through a systemic blood flow means that the remedy for ophthalmic diseases is administered in such a manner that it is transferred mainly by percutaneous permeation to the external ophthalmic tissue such as the conjunctiva, lacrimal tissue or cornea from the skin surface, on which the transdermal drug delivery system for treatment of ophthalmic diseases has been patched, and the efficacy thereof is developed before a part of the remedy for ophthalmic diseases reaches the external ophthalmic tissue through the systemic blood flow. Accordingly, it does not intend to exclude the fact that a part of the remedy for ophthalmic diseases is delivered to the external ophthalmic tissue through the systemic blood flow.

In the present invention, the skin surface including the front surface of the eyelid means skin surface of front surfaces of upper and lower eyelids, and skin surfaces of front surfaces of upper and lower eyelids and in the vicinity thereof. The front surface of the eyelid is covered with the skin, while the rear surface thereof is covered with the conjunctiva.

According to the transdermal drug delivery system for treatment of ophthalmic diseases of the present invention, the kind, amount and percutaneous absorptivity and the like of the remedy for ophthalmic diseases contained in the plaster layer are adjusted, whereby the remedy for ophthalmic diseases can be transferred in an amount effective for treatment to the external ophthalmic tissue in a relatively short period of time, and the efficacy thereof can be sustainedly developed. In the transdermal drug delivery system for treatment of ophthalmic diseases according to the present invention, the dose of the remedy for ophthalmic diseases per unit time can be controlled.

According to the transdermal drug delivery system for treatment of ophthalmic diseases of the present invention, the remedy can be supplied in an amount sufficient to develop the efficacy thereof by providing the preparation as a topical preparation that is administered from the skin surface including the front surface of the eyelid even when the remedy is low in percutaneous permeability. Even when the remedy is strong in stimulability, the drug efficacy can be reconciled with the suppression of skin stimulability by adjusting the percutaneous absorptivity of the remedy and the amount of the remedy penetrated into the skin.

In the transdermal drug delivery system for treatment of ophthalmic diseases according to the present invention, it is not always necessary to use a preservative when a pressure-sensitive adhesive is used as a base of the plaster. The transdermal drug delivery system for treatment of ophthalmic diseases according to the present invention can be administered during sleep. The present invention has been led to completion on the basis of these findings.

According to the present invention, there is provided a transdermal drug delivery system for treatment of ophthalmic diseases having a structure that a plaster layer containing a remedy for ophthalmic diseases and a pressure-sensitive adhesive is provided on a support, wherein the preparation is applied to a skin surface including a front surface of an eyelid to administer the remedy for ophthalmic diseases in the plaster layer to an external ophthalmic tissue including a conjunctiva, lacrimal tissue and cornea by percutaneous permeation substantially without being administered through a systemic blood flow, wherein the pressure-sensitive adhesive is a rubber-based pressure-sensitive adhesive, acrylic pressure-sensitive adhesive or silicone-based pressure-sensitive adhesive.

According to the present invention, there is also provided use of a transdermal drug delivery system for treatment of ophthalmic diseases having a structure that a plaster layer containing a remedy for ophthalmic diseases is provided on a support, comprising applying the transdermal drug delivery system for treatment of ophthalmic diseases to a skin surface including a front surface of an eyelid to transfer the remedy for ophthalmic diseases in the plaster layer to an external ophthalmic tissue by percutaneous permeation substantially without being administered through a systemic blood flow.

According to the present invention, there is further provided a method for transferring a remedy for ophthalmic diseases to an external ophthalmic tissue, comprising applying a transdermal drug delivery system for treatment of ophthalmic diseases having a structure that a plaster layer containing the remedy for ophthalmic diseases is provided on a support to a skin surface including a front surface of an eyelid to transfer the remedy for ophthalmic diseases in the plaster layer to the external ophthalmic tissue by percutaneous permeation substantially without being administered through a systemic blood flow.

BEST MODE FOR CARRYING OUT THE INVENTION

No particular limitation is imposed on the specific constitution of the transdermal drug delivery system for treatment of ophthalmic diseases according to the present invention so far as it has a structure that a plaster layer containing a remedy for ophthalmic diseases is provided on a support. Typical examples of the transdermal drug delivery system for treatment of ophthalmic diseases according to the present invention include cataplasms and pressure-sensitive adhesive tape preparations.

1. Cataplasm:

The cataplasm is a preparation comprising water, a water-soluble polymer, a wetting agent (humectant) and the like as base components for a plaster. The amount of water contained in a base is of the order of generally 20 to 70% by weight, preferably 30 to 60% by weight.

Examples of the water-soluble polymer include polyvinyl alcohol, polyacrylic acid, sodium polyacrylate, gelatin, agar, alginic acid, mannan, carboxymethyl cellulose, sodium carboxymethyl cellulose, methyl cellulose, sodium methyl cellulose, hydroxypropyl cellulose and sodium hydroxypropyl cellulose. These water-soluble polymers may be used either singly or in any combination thereof. The amount of the water-soluble polymer contained in the base is of the order of, generally 0.1 to 30% by weight, preferably 0.5 to 15% by weight.

Examples of the wetting agent include polyhydric alcohols such as polyethylene glycol, glycerol, sorbitol, maltitol, propylene glycol, 1,3-butanediol and reducing maltose syrup. The amount of the wetting agent contained in the base is of the order of, generally 10 to 60% by weight, preferably 20 to 50% by weight.

To the base of the cataplasm, as needed, a surfactant, crosslinking agent, filler, preservative, pH adjustor, antioxidant, ultraviolet absorbent, absorption enhancer, stabilizer, etc. may be added within limits permitting formulation of the preparation.

In order to formulate the cataplasm, a hydrous plaster with a remedy contained in the base is applied on to a support or releasable liner. More specifically, the transdermal drug delivery system of the cataplasm type can be prepared by a process in which the hydrous plaster is prepared by uniformly dispersing or dissolving the remedy for ophthalmic diseases in the base, and this hydrous plaster is spread on the support or releasable liner to bond and transfer the plaster under pressure on the support. The surface of the plaster layer is covered with a releasable liner.

2. Pressure-sensitive adhesive tape preparation:

The pressure-sensitive adhesive tape preparation is a transdermal drug delivery system of a structure that a pressure-sensitive adhesive layer containing a remedy for ophthalmic diseases is provided on a support. Since the pressure-sensitive adhesive tape preparation can be directly applied to a skin surface including an eyelid by the adhesion of the pressure-sensitive adhesive layer, it is easier in handling and application than the cataplasm. Examples of the pressure-sensitive adhesive used in the present invention include acrylic pressure-sensitive adhesives, rubber-based pressure-sensitive adhesives and silicone-based pressure-sensitive adhesives. Among these, the acrylic pressure-sensitive adhesives and rubber-based pressure-sensitive adhesives are preferred. The rubber-based pressure-sensitive adhesives are preferred because the kinds of a tackifier and other additives can be freely controlled.

As examples of the rubber-based pressure-sensitive adhesives, may be mentioned those comprising a rubbery elastic substance such as natural rubber, a styrene-isoprene-styrene block copolymer, polyisobutylene, polybutene or polyisoprene as an adhesive base.

The rubber-based pressure-sensitive adhesive is a composition obtained by adding a tackifier such as, for example, a rosin resin, terpene resin, coumarone-indene resin or petroleum resin to the rubbery elastic substance that is the adhesive base. To the adhesive base, as needed, may be added various kinds of additives, for example, a softening agent such as liquid polybutene, liquid polyisobutylene or mineral oil; a filler such as titanium oxide or zinc oxide; an antioxidant (stabilizer) such as butylhydroxytoluene or propyl gallate; and the like.

The tackifier is used in a proportion of generally 10 to 400 parts by weight, preferably 50 to 300 parts by weight, more preferably 70 to 200 parts by weight per 100 parts by weight of the rubbery elastic substance.

In the formulation of the pressure-sensitive adhesive tape preparation (also referred to as "patch preparation") making use of the rubber-based pressure-sensitive adhesive, is generally used a coating method of a solution of the pressure-sensitive adhesive, hot-melt method, calendering method or the like. In the coating method of the pressure-sensitive adhesive solution, the patch preparation is prepared by a process in which a solution containing the remedy for ophthalmic diseases and pressure-sensitive adhesive components in an organic solvent is coated on a releasable liner or support and dried. Examples of the organic solvent include toluene, ethyl acetate and hexane.

In the hot-melt method, the patch preparation is prepared by, for example, the following process. After the pressure-sensitive adhesive components other than the remedy for ophthalmic diseases are heated and stirred under purging with nitrogen to melt them, the temperature of the resultant melt is lowered, and the remedy component is then added to uniformly mix the respective components. The pressure-sensitive adhesive composition containing the remedy component is then spread on a releasable liner by a hot-melt coater, and a support is laminated thereon.

In the calendering method, the patch preparation is prepared by, for example, the following process. After the rubbery elastic substance is masticated, the temperature thereof is lowered, and the tackifier is then added to conduct kneading. After the temperature of the kneaded product is then further lowered, the softening agent is added to conduct kneading, and lastly the remedy component is added to conduct kneading, thereby preparing a pressure-sensitive adhesive composition. This pressure-sensitive adhesive composition is spread on a releasable liner, and a support is laminated thereon. Temperature conditions, kneading time and the like may be suitably changed according to the kind of the rubbery elastic substance, the formulation of the pressure-sensitive adhesive composition, and the like. In general, the pressure-sensitive adhesive composition is coated on the releasable liner. However, the composition may be coated on the support, and the releasable liner may be laminated as a coating material as needed.

Among the rubber-based pressure-sensitive adhesives, are preferred those obtained by using a styrene-isoprene-styrene block copolymer (hereinafter may be abbreviated as "SIS" in some cases) as a main adhesive base and, as needed, blending other rubbery elastic substances or the like together with the tackifier from the viewpoints of stability, percutaneous absorptivity and percutaneous permeability of the remedy, tackiness, and the like.

The acrylic pressure-sensitive adhesives include (co)polymers of at least one alkyl (meth)acrylate and copolymers of an alkyl (meth)acrylate and a functional monomer and/or vinyl ester monomer copolymerizable with this ester.

The alkyl (meth)acrylate is used in a proportion of generally 50 to 100% by weight, preferably 60 to 97% by weight. The functional monomer is used in a proportion of generally 0 to 30% by weight, preferably 2 to 10% by weight. The vinyl ester monomer is used in a proportion of generally 0 to 40% by weight, preferably 5 to 30% by weight.

The number of carbon atoms of the alkyl group moiety in the alkyl (meth)acrylate is preferably within a range of 4 to 10. Examples of such alkyl (meth)acrylates include butyl acrylate, octyl acrylate, 2-ethylhexyl acrylate, nonyl acrylate and isononyl acrylate. Examples of the functional monomer include (meth)acrylic acids having a functional group. specific examples thereof include acrylic acid, methacrylic acid and 2-hydroxyethylacrylic acid. Examples of the vinyl ester monomer include vinyl acetate and vinyl laurate.

The acrylic pressure-sensitive adhesive is generally synthesized by solution polymerization, suspension polymerization and emulsion polymerization. A patch preparation may be prepared by dispersing or dissolving the remedy for ophthalmic diseases in a solution or emulsion of the acrylic pressure-sensitive adhesive, applying the resultant solution or dispersion on to a releasable liner or support and drying it. This acrylic pressure-sensitive adhesive is preferably crosslinked by adding a small amount of a crosslinking agent.

Examples of the silicone-based pressure-sensitive adhesives include those comprising bifunctional or trifunctional polysiloxane, or the like as a main component. A patch preparation may be prepared by dispersing or dissolving the remedy for ophthalmic diseases in the silicone-based pressure-sensitive adhesive or a solution thereof, applying or spreading the resultant solution or dispersion on to a releasable liner or support.

3. Support:

The support preferably has flexibility to an extent that it can be brought into close contact with a skin surface including a front surface of a rolling eyelid. The support is preferably such that it does not absorb the remedy, and the remedy is not released from the side of the support. As specific examples of the support, may be mentioned nonwoven fabrics, fabrics, films (including sheets), porous bodies, foamed bodies, paper, and composite materials obtained by laminating a film on a nonwoven fabric or fabric. However, the support is not limited thereto.

Examples of a material for the nonwoven fabric used as the support include polyolefin resins such as polyethylene and polypropylene; polyester resins such as polyethylene terephthalate, polybutylene terephthalate and polyethylene naphthalate; and besides rayon, polyamide, poly(ester ether), polyurethane, polyacrylic resins, polyvinyl alcohol, styrene-isoprene-styrene copolymers, and styrene-ethylene-propylene-styrene copolymers. As examples of a material for the fabric, may be mentioned cotton, rayon, polyacrylic resins, polyester resins and polyvinyl alcohol. However, the materials are not limited thereto.

Examples of a material for the film used as the support include polyolefin resins such as polyethylene and polypropylene; polyacrylic resins such as polymethyl methacrylate and polyethyl methacrylate; polyester resins such as polyethylene terephthalate, polybutylene terephthalate and polyethylene naphthalate; and besides cellophane, polyvinyl alcohol, ethylene-vinyl alcohol copolymers, polyvinyl chloride, polystyrene, polyurethane, polyacrylonitrile, fluororesins, styrene-isoprene-styrene copolymers, styrene-butadiene rubber, polybutadiene, ethylene-vinyl acetate copolymers, polyamide, and polysulfone. However, the materials are not limited thereto.

Examples of paper include impregnated paper, coated paper, wood free paper, kraft paper, Japanese paper, glassine paper and synthetic paper. As examples of the composite materials, may be mentioned composite materials obtained by laminating the above-described film on the above-described nonwoven fabric or fabric.

4. Remedy for ophthalmic diseases:

In the present invention, remedies for ophthalmic diseases are used as drugs. As the remedies for ophthalmic diseases, may be used various kinds of drugs used in ophthalmic solutions, ophthalmic ointments and the like. Specific examples (drug efficacy classifications) of such remedies for ophthalmic diseases are indicated together with examples of diseases for which they are efficacious as needed and include antiviral agents (keratitis caused by herpes simplex), antibacterial agents (infectious diseases: conjunctivitis, blepharitis, corneal tumor and dacryocystitis), anti-mycotic agents, antiallergic agents (allergic conjunctivitis, pollinosis and vernal conjunctivitis), anti-inflammatory agents (conjunctivitis, superficial keratitis, marginal blepharitis and scleritis), nonsteroidal anti-inflammatory agents (allergic conjunctivitis), anti-inflammatory-analgesic agents, anti-inflammatory enzymatic agents (chronic conjunctivitis), antibiotics (infectious diseases: trachoma, conjunctivitis, blepharitis, marginal blepharitis, keratitis, hordeolum, corneal ulcer, tarsadenitis and dacryocystitis), sulfa agents (trachoma, conjunctivitis, blepharitis, marginal blepharitis, corneal ulcer and keratitis), synthetic penicillin (infectious diseases), remedies for glaucoma, remedies for cataract, miotics, mydriatics, topical astringents, vasopressors, preventives for rise in ocular tension, remedies for ocular hypertension, surface anesthetics, $\alpha$1-blockers (glaucoma and ocular hypertension), $\beta$-blockers (glaucoma and ocular hypertension), $\beta$1-blockers (glaucoma and ocular hypertension), carbonic anhydrase inhibitors, topical selective H1-blockers (allergic conjunctivitis), adrenal cortical hormone (nosotropic method for inflammatory diseases of external and anterior ocular segments), vitamin B12 (asthenopia), coenzyme type vitamin B2 (keratitis and blepharitis), anticholinesterase agents (glaucoma, accommodative esotropia and myasthenia gravis), and organic iodine preparations (central retinitis and the like).

Among these remedies for ophthalmic diseases, antibacterial agents, antiallergic agents and nonsteroidal anti-inflammatory agents (antiphlogistics) are particularly preferred. As the object diseases, are preferred ocular infection, allergic conjunctivitis, pollinosis and vernal conjunctivitis. The transdermal drug delivery system for treatment of ophthalmic diseases according to the present invention may also be preferably used in prevention and treatment for postoperative inflammation and postoperative infection.

As examples of specific names of drugs used in ophthalmic solutions, ophthalmic ointments, etc., may be mentioned acyclovir, acitazanolast hydrate, azulene, anthranilic acid, ascorbic acid, amlexanox, isopropyl unoprostone, idoxuridine, ibudilast, indomethacin, epinephrine, erythromycin, lysozyme chloride, apraclonidine hydrochloride, oxybuprocaine hydrochloride, carteolol hydrochloride, cyclopentolate hydrochloride, dipivefrin hydrochloride, cefmenoxim hydrochloride, dorzolamide hydrochloride, pilocarpine hydrochloride, phenylephrine hydrochloride, bunazosin hydrochloride, betaxolol hydrochloride, befunolol hydrochloride, levocabastine hydrochloride, levobunolol hydrochloride, lomefloxacin hydrochloride, ofloxacin, carbachol, dipotassium glycyrrhitinate, glutathione, sodium cromoglycate, chloramphenicol, hydrocortisone acetate, prednisolone acetate, cyanocobalamin, diclofenac sodium, distigmine bromide, homatropine hydrobromide, silver nitrate, naphazoline nitrate, calcium diiodostearate, sulfisoxazole, sulbenicillin sodium, dexamethasone, tobramycin, tranilast, tropicamide, nipradilol, norfloxacin, pimaricin, pirenoxine, ketotifen fumarate, pranoprofen, flavin-adenine dinucleotide, fluorometholone, predonisolone, bromofenac sodium hydrate, pemirolast potassium, helenien, timolol maleate, miopin, dexamethasone sodium m-sulfobenzoate, ecothiopate iodide, latanoprost, lidocaine hydrochloride, atropine sulfate, gentamicin sulfate, sisomicin sulfate, dibekacin sulfate, micronomicin sulfate, dexamethasone sodium phosphate, betamethasone disodium phosphate and levofloxacin.

Among these remedies for ophthalmic diseases, drugs each composed of a compound having a molecular weight of at most 1,000 are preferred from the viewpoints of percutaneous absorptivity and percutaneous permeability. Among these remedies for ophthalmic diseases, antibacterial agents, antiallergic agents and nonsteroidal anti-inflammatory agents having a molecular weight of at most 1,000 are more preferred. The molecular weights of these remedies for ophthalmic diseases are more preferably at most 800, particularly preferably at most 600 or 500.

Among the above-described remedies for ophthalmic diseases, ketotifen fumarate (antiallergic agent, antihistaminic; molecular weight: 425.5) and diclofenac sodium (nonsteroidal anti-inflammatory agent; molecular weight: 318.13) are particularly preferred from the viewpoints of percutaneous absorptivity, percutaneous permeability, drug efficacy and the like.

5. Solubilizing agent:

When the remedy for ophthalmic diseases is required to make easy to be dissolved in the pressure-sensitive adhesive or the base of the cataplasm in the present invention, a solubilizing agent may be used upon the preparation of a coating fluid or coating composition containing the respective components. Examples of the solubilizing agent include crotamiton, ethanol, urea, water-soluble organic amines, fatty acid esters of propylene glycol, 1-menthol and peppermint oil. The solubilizing agents may be used either singly or in any combination thereof.

6. Percutaneous absorption enhancer (percutaneous permeation enhancer):

In the present invention, a percutaneous absorption enhancer may be used for accelerating the percutaneous absorption of the remedy for ophthalmic diseases. In the present invention, the percutaneous absorption enhancer may also be referred to as a percutaneous permeation enhancer because it accelerates not only the percutaneous absorption of the remedy but also the percutaneous permeation of the remedy.

Examples of the percutaneous absorption enhancer include aliphatic alcohols, fatty acids, fatty acid esters, alcohol amines, polyhydric alcohol alkyl ethers, polyoxyethylene alkyl ethers, glycerides (i.e., fatty acid esters of glycerol), middle-chain fatty acid esters of polyhydric alcohols, lactic acid alkyl esters, dibasic acid alkyl esters, acylated amino acids, and pyrrolidone and derivatives thereof. However, the enhancers are not limited thereto. These percutaneous absorption enhancers may be used either singly or in any combination thereof.

As the aliphatic alcohols, are preferred, for example, saturated or unsaturated higher alcohols having 12 to 22 carbon atoms, such as oleyl alcohol and lauryl alcohol. Examples of the fatty acids include linolic acid, oleic acid, linolenic acid, stearic acid, isostearic acid and palmitic acid.

Examples of the alcohol amines include triethanolamine, triethanolamine hydrochloride and diisopropanolamine.

Examples of the fatty acid esters include isopropyl myristate, diisopropyl adipate and isopropyl palmitate.

Examples of the polyhydric alcohol alkyl ethers include alkyl ethers of polyhydric alcohols such as glycerol, ethylene glycol, propylene glycol, 1,3-butylene glycol, diglycerol, polyglycerol, diethylene glycol, polyethylene glycol, dipropylene glycol, polypropylene glycol, sorbitan, sorbitol, isosorbide, methyl glucoside, oligosaccharides and reducing oligosaccharides. The number of carbon atoms of the alkyl group moiety in the polyhydric alcohol alkyl ethers is preferably 6 to 20.

The polyoxyethylene alkyl ethers are preferably polyoxyethylene alkyl ethers, in which the number of carbon atoms.of the alkyl group moiety is 6 to 20, and the number of repeating units ($-O-CH_2CH_2-$) of the polyoxyethylene chain is 1 to 9. Specific examples of such polyoxyethylene alkyl ethers include polyoxyethylene lauryl ether, polyoxyethylene cetyl ether, polyoxyethylene stearyl ether and polyoxyethylene oleyl ether.

As the glycerides, are preferred glycerol esters of fatty acids having 6 to 18 carbon atoms. The glycerides are divided into monoglycerides, diglycerides and triglycerides according to the number of fatty acids bonded. However, all the glycerides may be used in the present invention. They may also be mixtures (for example, mixtures of mono- and diglycerides) thereof. Examples of preferable fatty acid components forming the glycerides include octanoic acid, decanoic acid, dodecanoic acid, tetradecanoic acid, hexadecanoic acid, octadecanoic acid (i.e., stearic acid) and oleic acid.

Besides, various kinds of percutaneous absorption enhancers such as lactic acid, tartaric acid, 1,2,6-hexanetriol, benzyl alcohol, lanoline, potassium hydroxide (KOH) and tris(hydroxymethyl)aminomethane may be suitably used.

Among these percutaneous absorption enhancers (percutaneous permeation enhancers), aliphatic higher alcohols such as lauryl alcohol; fatty acids such as isostearic acid; alcohol amines such as diisopropanolamine; fatty acid esters such as isopropyl myristate and isopropyl palmitate; polyoxyethylene alkyl ethers such as polyoxyethylene oleyl ether; the other compounds such as KOH and tris(hydroxymethyl)aminomethane; and mixtures of two or more compounds thereof are preferred.

7. Proportions of respective components used:

The remedy for ophthalmic diseases is used in a proportion of generally 0.1 to 60 parts by weight, preferably 0.3 to 20 parts by weight per 100 parts by weight of the plaster base such as the pressure-sensitive adhesive. If the proportion of the remedy for ophthalmic diseases contained is too low, it is difficult to achieve sustainedly sufficient drug efficacy. If the proportion is too high, crystals may be deposited to lower adhesion in some cases.

The solubilizing agent is used in a proportion of generally 0 to 60 parts by weight, preferably 0 to 20 parts by weight per 100 parts by weight of the pressure-sensitive adhesive. The percutaneous absorption enhancer (percutaneous permeation enhancer) is used in a proportion of generally 1 to 50 parts by weight, preferably 2 to 40 parts by weight per 100 parts by weight of the plaster base such as the pressure-sensitive adhesive. In the plaster layer such as a pressure-sensitive adhesive layer, as needed, various kinds of additives known in this technical field may be contained so far as they impede the efficacy of the remedy for ophthalmic diseases and the tackiness of the pressure-sensitive adhesive.

8. Preparation process for transdermal drug delivery system:

The transdermal drug delivery system for treatment of ophthalmic diseases according to the present invention may be prepared in accordance with the already described process. However, a more preferable preparation example thereof will be described more specifically herein. The transdermal drug delivery system for treatment of ophthalmic diseases according to the present invention has the structure that the plaster layer containing the remedy for ophthalmic diseases is provided on the support. A releasable liner is generally provided on the plaster layer such as the pressure-sensitive adhesive layer. The releasable liner is used for the purpose of protecting the plaster layer. Examples thereof include those obtained by subjecting one side of polyethylene-coated wood free paper, polyolefin-coated glassine paper, a polyethylene terephthalate (polyester) film, a polypropylene film or the like to a silicone treatment.

When the transdermal drug delivery system for treatment of ophthalmic diseases according to the present invention is a cataplasm, it may be prepared by applying a hydrous plaster with a drug contained in a base to a support or releasable liner. When the transdermal drug delivery system for treatment of ophthalmic diseases according to the present invention is a patch preparation (pressure-sensitive adhesive tape preparation), it may be prepared in accordance with the solvent coating method, hot-melt method, calendering method or the like that is a general preparation method for the patch preparation.

The transdermal drug delivery system for treatment of ophthalmic diseases according to the present invention is preferably a pressure-sensitive adhesive tape type preparation making use of a rubber-based pressure-sensitive adhesive. According to the solvent coating method, an organic solvent such as n-hexane, toluene or ethyl acetate is placed in a metal kettle lined with glass, various rubbery elastic substances, a tackifier, an antioxidant, etc. are added into this kettle, and the contents are stirred for 2 to 10 hours, preferably 3 to 7 hours until they are uniformly dissolved. To this solution of the pressure-sensitive adhesive, a prescribed amount of the remedy for ophthalmic diseases, and optionally the solubilizing agent, percutaneous absorption enhancer, etc. are added, and the resultant mixture is continuously stirred for 10 to 120 minutes. A prescribed amount of a coating fluid obtained in such a manner is coated on a support by means of a coater such as a knife coater, comma coater or reverse coater. After the coating, the coated support is placed for about 1 to 10 minutes in an atmosphere controlled at a fixed temperature of 40 to 120°C to vaporize out the organic solvent. The drying conditions are suitably set according to the kind of the organic solvent and the thickness of the coating layer. A releasable liner such as a polyester film or polyethylene-coated wood free paper subjected to a silicone treatment is laminated on the surface of the pressure-sensitive adhesive layer thus formed, and the thus-obtained laminate is cut to proper size to provide a patch preparation. After the coating fluid is coated on one side of the releasable liner and dried, the support may also be laminated on the surface of the pressure-sensitive adhesive layer thus formed.

When the pressure-sensitive adhesive is capable of hot-melt coating, the adhesive may be subjected to hot-melt coating. In the hot-melt method, after the pressure-sensitive adhesive components other than the remedy component are heated and stirred at a temperature of 100 to 150°C under purging with nitrogen to melt them, the temperature of the resultant melt is lowered to a temperature of 100 to 120°C, and the remedy component is then added to uniformly mix the respective components. The pressure-sensitive adhesive composition containing the remedy component is then spread on a releasable liner by a hot-melt coater, and a support is laminated thereon, thereby preparing a patch preparation. The temperature conditions and the like are preferably set to optimum ranges according to the kind of the pressure-sensitive adhesive base, and the like.

In the case of the calendering method, for example, after the rubbery elastic substance is masticated at 130°C for about 5 to 20 minutes, the temperature thereof is lowered to about 100 to 120°C, and the tackifier is added to conduct kneading for about 5 to 10 minutes. After the temperature of the kneaded product is then lowered to about 70 to 90°C, the softening agent is added to conduct kneading for about 5 to 10 minutes, and lastly the remedy for ophthalmic diseases, solubilizing agent, percutaneous absorption enhancer and the like are added to conduct kneading for about 5 to 10 minutes, thereby obtaining a drug-containing plaster. This plaster is spread in a thickness of 0.1 mm on a polyester film subjected to the silicone treatment, and the support is then laminated on the pressure-sensitive adhesive layer thus formed, whereby a patch preparation can be prepared. The temperature, kneading time and the like may be suitably set to preferable ranges according to the kind of the pressure-sensitive adhesive base used, and the like.

The transdermal drug delivery system for treatment of ophthalmic diseases according to the present invention is also preferably a pressure-sensitive adhesive tape type preparation making use of an acrylic pressure-sensitive adhesive. As described above, a patch preparation can be prepared by dispersing or dissolving the remedy for ophthalmic diseases in a solution or emulsion of the acrylic pressure-sensitive adhesive, applying the resultant solution or dispersion on to a releasable liner or support and drying it.

The thickness of the plaster layer is generally 0.3 to 2.0 mm for the cataplasm and generally 10 to 300 $\mu$m for the pressure-sensitive adhesive type preparation.

9. <u>Transdermal drug delivery system for treatment of ophthalmic diseases:</u>

The transdermal drug delivery system for treatment of ophthalmic diseases according to the present invention is used for applying it to a skin surface containing a front surface of an eyelid to percutaneously administer the remedy for ophthalmic diseases in the plaster layer to an ophthalmic topical tissue. The skin surface including the front surface of the eyelid means a front surface (skin surface) of an upper eyelid, a lower eyelid or both eyelids, or skin surfaces of these eyelids and skin surfaces around them.

Therefore, the transdermal drug delivery system for treatment of ophthalmic diseases according to the present invention preferably has a form capable of being applied along a skin surface of the upper eyelid, the lower eyelid or both eyelids. Specific examples of such a form include forms such as a rectangle, an ellipse, a crescent, a circle, a horseshoe and a ring along the form of the front surface(s) of the eyelid(s).

As a preferred embodiment of "the plaster layer containing the remedy for ophthalmic diseases" making up the transdermal drug delivery system for treatment of ophthalmic diseases according to the present invention, may be mentioned a pressure-sensitive adhesive layer containing the following components.

(1) Rubber-based pressure-sensitive adhesive layer:

     i) SIS: 100 parts by weight,
     ii) Tackifier: 10 to 400 parts by weight,
     iii) Percutaneous absorption enhancer: 1 to 50 parts by weight,
     iv) Remedy for ophthalmic diseases: 0.1 to 60 parts by weight.

(2) Acrylic pressure-sensitive adhesive layer:

     i) Acrylic (co)polymer: 100 parts by weight,
     ii) Percutaneous absorption enhancer: 1 to 50 parts by weight,

iii) Remedy for ophthalmic diseases: 0.1 to 60 parts by weight.

As the remedies for ophthalmic diseases contained in these pressure-sensitive adhesive layers, are preferred the above-described drugs composed of a compound having a molecular weight of at most 1,000, with antibacterial agents, antiallergic agents and nonsteroidal anti-inflammatory agents having a molecular weight of at most 1,000 being more preferred. Among these remedies for ophthalmic diseases, ketotifen fumarate and diclofenac sodium are particularly preferred from the viewpoints of percutaneous absorptivity, percutaneous permeability, drug efficacy and the like.

As examples of the percutaneous absorption enhancer, are preferred the above-described aliphatic higher alcohols such as lauryl alcohol; fatty acids such as isostearic acid; alcohol amines such as diisopropanolamine; fatty acid esters such as isopropyl myristate and isopropyl palmitate; polyoxyethylene alkyl ethers such as polyoxyethylene oleyl ether; the other compounds such as KOH and tris(hydroxymethyl)aminomethane; and mixtures of two or more compounds thereof.

According to the transdermal drug delivery system for treatment of ophthalmic diseases according to the present invention, the remedy for ophthalmic diseases in a plaster layer can be administered by percutaneous permeation to an ophthalmic topical tissue substantially without being administered through a systemic blood flow by applying the preparation to a skin surface including a front surface of an eyelid.

As described above, the fact that the remedy for ophthalmic diseases in the plaster layer in the transdermal drug delivery system for treatment of ophthalmic diseases according to the present invention is administered by percutaneous permeation to the ophthalmic topical tissue substantially without being administered through a systemic blood flow means that the remedy for ophthalmic diseases is administered in such a manner that it is transferred mainly by percutaneous permeation to an external ophthalmic tissue such as the conjunctiva, lacrimal tissue or cornea from a skin surface, on which the transdermal drug delivery system for treatment of ophthalmic diseases has been patched, and the efficacy thereof is developed before a part of the remedy for ophthalmic diseases reaches the ophthalmic topical tissue through the systemic blood flow.

More specifically, when the transdermal drug delivery system for treatment of ophthalmic diseases according to the present invention is applied to the skin surface including the front surface of the eyelid, the amount (unit: $\mu$g/g·tissue) of the remedy transferred to an external ophthalmic tissue under the application within 8 hours after the application amounts to generally at least twice, preferably at least 5 times, more preferably at least 7 times, particularly preferably at least 8 times as much as the amount of the remedy transferred to the external ophthalmic tissue through the systemic blood flow.

When the transdermal drug delivery system for treatment of ophthalmic diseases according to the present invention is applied to the skin surface including the front surface of the eyelid, the amount (unit: $\mu$g/g·tissue) of the remedy transferred to an external ophthalmic tissue under the application within 8 hours after the application amounts to at least 50 times, further at least 100 times, as much as the amount of the remedy transferred to the external ophthalmic tissue through the systemic blood flow as compared with the case where the transdermal drug delivery system for treatment of ophthalmic diseases is applied to a skin surface of a part distant from the skin surface around the eye, such as the back.

When the transdermal drug delivery system for treatment of ophthalmic diseases according to the present invention is applied to a skin surface including a front surface of an eyelid of one eye, the amount (unit: $\mu$g/g·tissue) of the remedy transferred to an external ophthalmic tissue under the application within 8 hours after the application amounts to at least 10 times as much as the amount of the remedy transferred to an external ophthalmic tissue such as the conjunctiva of the other eye.

The measuring method and results of these transferred amounts of the remedy for ophthalmic diseases will be describe in detail in the following Examples.

EXAMPLES

The present invention will hereinafter be described more specifically by the following Examples and Comparative Examples.

[Example 1]

A pressure-sensitive adhesive solution (coating fluid) having a solid content of 40% by weight was obtained by dissolving 40.5 g of a styrene-isoprene-styrene block copolymer (product of JSR Corporation, trade name "SIS5000") as a rubbery elastic substance, 40.5 g of a terpene resin (product of YASUHARA CHEMICAL CO., LTD., trade name "YS Resin 1150N") as a tackifier, 1 g of butylhydroxytoluene as an antioxidant, 10 g of ketotifen fumarate that is an antiallergic agent for treatment of ophthalmic diseases, and 3 g of lauryl alcohol and 5 g of diisopropanolamine as absorption enhancers in 150 g of toluene. This coating fluid was coated on release paper so as to give a dry coat

thickness of 40 $\mu$m. After drying, a support (polyester film having a thickness of 12 $\mu$m) was laminated to provide a patch preparation.

[Example 2]

Four hundred grams of a styrene-isoprene-styrene block copolymer (product of JSR Corporation, trade name "Cariflex TR-1107") as a rubbery elastic substance, 400 g of a terpene resin (YS Resin 1150N) as a tackifier, 125 g of liquid paraffin as a softening agent, 5 g of diclofenac sodium that is a nonsteroidal anti-inflammatory agent for treatment of ophthalmic diseases, and 60 g of isostearic acid as an absorption enhancer were uniformly mixed by kneading using a heating kneader. After the kneading, the mixture was spread on a silicone surface of a releasable liner, on one surface of which had been subjected to a silicone treatment, by means of a calender so as to give a thickness of 200 $\mu$m, and a support (polyester film having a thickness of 12 $\mu$m) was then laminated thereon to provide a patch preparation.

[Comparative Example 1]

A commercially available ophthalmic solution (Zaditen ophthalmic solution 0.05%, product of Sankyo-Novartis Co., Ltd.) containing 0.05% of ketotifen fumarate was used.

[Comparative Example 2]

A commercially available ophthalmic solution (Diclod ophthalmic solution 0.1%, product of WAKAMOTO PHARMACEUTICAL CO., LTD.) containing 0.1% of diclofenac sodium was used.

<Test for transferability to the conjunctiva>

A test preparation cut in a rectangle of 1 x 4 cm was applied to a shaved portion of a lower eyelid of a rabbit. The test preparation was removed after 1 hour and 12 hours, and the skin of the eyelid portion was separated under anesthesia to take out the conjunctiva. The amount of the drug contained in the conjunctiva was determined by high performance liquid chromatography (HPLC). With respect to the ophthalmic solution, the same operation was conducted after one drop of the ophthalmic solution was applied to the eye of a rabbit. The results of the test for transferability to the conjunctiva are shown in Tables 1 and 2.

Table 1

| Results of the test for transferability to the conjunctiva | | | |
|---|---|---|---|
| No. | | Example 1 | Comp. Example 1 |
| Preparation form | | Percutaneous absorption type | Ophthalmic solution |
| Drug | | Ketotifen fumarate | Ketotifen fumarate |
| Amount transferred to conjunctiva ($\mu$g/g) | 1 hr | 0.06 $\pm$ 0.02 | 0.03 $\pm$ 0.01 |
| | 12 hrs | 0.10 $\pm$ 0.02 | 0.02 $\pm$ 0.01 |
| n = 5, mean $\pm$ SE | | | |

Table 2

| Results of the test for transferability to the conjunctiva | | | |
|---|---|---|---|
| No. | | Example 2 | Comp. Example 2 |
| Preparation form | | Percutaneous absorption type | Ophthalmic solution |
| Drug | | Diclofenac sodium | Diclofenac sodium |
| Amount transferred to conjunctiva ($\mu$g/g) | 1 hr | 0.10 $\pm$ 0.03 | 0.06 $\pm$ 0.01 |
| | 12 hrs | 0.05 $\pm$ 0.01 | N.D. |
| n = 5, mean $\pm$ SE | | | |

As shown in Table 1, the transdermal drug delivery system for treatment of ophthalmic diseases according to the present invention (Example 1) was recognized to have high transferability of ketotifen fumarate to the conjunctiva over a long period of time. On the other hand, it was demonstrated that the ophthalmic solution (Comparative Example 1) is rapidly washed out by tears, and only a little amount of the drug remains after 1 hour from the administration, and so potency over a long period of time cannot be expected.

As shown in Table 2, it is understood that the transdermal drug delivery system for treatment of ophthalmic diseases according to the present invention (Example 2) is recognized to have high transferability of diclofenac sodium to the conjunctiva and develops potency to various inflammatory ophthalmic diseases.

From the above, it is understood that even a transdermal drug delivery system generally low in percutaneous permeability of the drug can percutaneously administer the drug in an amount effective for treatment by applying it to the skin surface of an eyelid and can sustain its efficacy over a long period of time. Accordingly, the transdermal drug delivery systems according to the present invention can be applied to treatment of ophthalmic diseases. The remedy for ophthalmic diseases is administered in the form of a transdermal drug delivery system, whereby high sustainability of drug efficacy that is not found in any ophthalmic solution, and a new administration mode such as application during sleep, which cannot be done in the conventional preparations, can be provided.

[Example 3]

In accordance with the formulation shown in the following Table 3, 0.0015 g of a crosslinking agent (product of NIPPON CARBIDE INDUSTRIES CO., INC., trade name "NISSETSU CK-401"; metal chelating agent), 0.3 g of ketotifen fumarate, and 0.6 g of polyoxyethylene oleyl ether and 0.6 g of isopropyl myristate as percutaneous absorption enhancers were added to 3.713 g (solids: 1.485 g) of an acrylic pressure-sensitive adhesive [product of NIPPON CARBIDE INDUSTRIES CO., INC., trade name "NISSETSU PE-300"; alkyl (meth)acrylate-vinyl acetate copolymer; pressure-sensitive adhesive solution having a solid content of 40% by weight (ethyl acetate/toluene mixed solvent)] to prepare a coating fluid having a concentration of 57.3% by weight. This coating fluid was coated on release paper so as to give a dry coat thickness of 80 $\mu$m. After drying, a support (polyester film having a thickness of 12 $\mu$m) was laminated to provide a patch preparation.

Table 3

| Composition | |
|---|---|
| Ketotifen fumarate | 0.3 g (10 wt.%) |
| Polyoxyethylene oleyl ether | 0.6 g (20 wt.%) |
| Isopropyl myristate | 0.6 g (20 wt.%) |
| Acrylic pressure-sensitive adhesive; "NISSETSU PE-300", product of NIPPON CARBIDE INDUSTRIES CO., INC. | 1.485 g (solids) |
| Crosslinking agent; "NISSETSU CK-401", product of NIPPON CARBIDE INDUSTRIES CO., INC. | 0.0015 g |

The patch preparation (transdermal drug delivery system for treatment of ophthalmic diseases) obtained above was used to test the transferability of ketotifen fumarate that is a drug to the conjunctiva in accordance with the following method.

<Test for transferability to the conjunctiva>

1. Testing method:

1) Animal

As animals, Japanese white male rabbits weighing about 2 kg were purchased from Fukuzaki Rabbit Rearing Association and respectively reared under conditions of a temperature of 23 $\pm$ 3°C and a humidity of 55 $\pm$ 10% in a rearing room in a conventional zone.

2) Pretreatment of animal

Rabbits shaved in advance were used to apply a transdermal drug delivery system thereto. The shaving was conducted on the peripheral portions of the eyes of each rabbit and the back thereof under mixed anesthesia of ketamine and xylazine by means of a hair clipper and a shaver carefully to avoid injuring the skin.

3) Administration

The system of ketotifen preparation was applied to the skin of the upper and lower eyelids and the back skin of each rabbit each by 4 cm$^2$.

In the case of the skin of the upper and lower eyelids, pieces of the transdermal drug delivery system cut in the size of 1 cm x 2 cm = 2 cm$^2$ were respectively applied on to the upper and lower eyelids by 2 cm$^2$.

In the case of the back skin, a piece of the transdermal drug delivery system cut in the size of 2 cm x 2 cm = 4 cm$^2$ was applied on to the back skin.

4) Collection of ophthalmic tissues

After a tear fluid was collected by a capillary at the following predetermined points of time, the rabbits were euthanized with an excess amount of a sodium pentobarbital solution. After anterior ocular segments were washed with physiological saline, the eyes were enucleated in a state the conjunctivae had been attached, and the conjunctivae were then collected.

Collecting time: 4, 8 and 24 hours.

5) Pretreatment of ophthalmic tissue sample

Conjunctiva: One milliliter of sodium dihydrogenphosphate buffer was added to finely cut the conjunctiva. After 4 mL of acetonitrile was added to conduct shaking for 10 minutes at 300 rpm, centrifugation was conducted for 10 minutes at 3,000 rpm. After 4 mL of supernatant was taken out in another test tube and dried to solids under reduced pressure, it was re-dissolved in a mobile phase of HPLC. The solution was filtered through a membrane filter (0.22 $\mu$m), and a filtrate was used as a sample for HPLC measurement.

Tear fluid: After 150 $\mu$L of a mobile phase of HPLC was added to stir the resultant mixture, the mixture was used as a sample for HPLC measurement.

6) Concentration measurement

High performance liquid chromatography was used to determine the concentration of ketotifen fumarate under the following HPLC conditions.

| | |
|---|---|
| Detector: | Ultraviolet absorptiometer (measurement wavelength: 300 nm) |
| Column: | Capcell pack C18MGS 5 $\mu$m, 4.5 x 250 mm, manufactured by Shiseido Co., Ltd.; Guard Column (TOSOH, 80 Ts) |
| Column temperature: | Fixed temperature about 40°C |
| Mobile phase: | 0.1 M tris(hydroxymethyl)aminomethane |
| buffer (pH 9): | acetonitrile = 30:70 |
| Flow rate: | 1.0 mL/min |
| Injection volume: | 50 $\mu$m |

The results of the test for transferability of ketotifen fumarate to the conjunctiva making use of the transdermal drug delivery system for treatment of ophthalmic diseases prepared in Example 3 are shown in Table 4.

Table 4

| Results of the test for transferability to the conjunctiva | | | |
|---|---|---|---|
| Time | Application to upper and lower eyelid parts | | Application to the back |
| | Conjunctivae of applied eye | Conjunctivae of the other eye | Conjunctivae of both eyes |
| 4 | 4.44 | 0.39 | 0.01 |
| 8 | 2.95 | 0.03 | 0.02 |

(continued)

| Results of the test for transferability to the conjunctiva | | | |
|---|---|---|---|
| Time | Application to upper and lower eyelid parts | | Application to the back |
| | Conjunctivae of applied eye | Conjunctivae of the other eye | Conjunctivae of both eyes |
| 24 | 0.13 | 0.01 | 0.01 |
| Unit: [μg/g·tissue] | | | |

As apparent from the results shown in Table 4, it is understood that when the transdermal drug delivery system for treatment of ophthalmic diseases according to the present invention is applied to the skin of the back of the subject animal, the amount of the drug (ketotifen fumarate) penetrated into the skin surface under the application, absorbed in an intraepithelial blood capillary and reached the conjunctivae of both eyes through the systemic blood flow from the blood capillary is at the level of about 0.01 to 0.02 μg/g even when 4 hours, 8 hours and 24 hours have elapsed from the application, namely, the amount of the drug transferred to the ophthalmic topical tissues (conjunctivae of the external ophthalmic tissues) through the systemic blood flow is extremely little.

On the contrary, it is understood that when the transdermal drug delivery system for treatment of ophthalmic diseases according to the present invention is applied to the skin surfaces of the upper and lower eyelids, the drug is transferred to the conjunctivae under the application at a concentration as high as 4.44 μg/g after 4 hours from the application, and the amount transferred retain high levels of 2.95 μg/g after 8 hours and 0.13 μg/g after 24 hours.

On the other hand, the amount of the drug transferred to the conjunctivae of the eye (the other eye), to which no transdermal drug delivery system for treatment of ophthalmic diseases according to the present invention has been applied, indicates a relatively high level of 0.39 μg/g after 4 hours, but is only a level to the extent of about 10% by weight of the amount transferred to the conjunctivae under the application, i.e., 4.44 μg/g. It is also understood that the amount of the drug transferred is markedly reduced to 0.03 μg/g after 8 hours and 0.01 μg/g after 24 hours, and so the sustainability of the drug efficacy is poor.

The above-described experimental results clearly indicate that the transdermal drug delivery system for treatment of ophthalmic diseases according to the present invention is such that the remedy for ophthalmic diseases in the plaster layer can be administered by percutaneous permeation to the ophthalmic topical tissues substantially without being administered through the systemic blood flow. More specifically, the above-described experimental results indicate that there is a marked difference in the concentration of the drug in the conjunctivae between the case where the transdermal drug delivery system for treatment of ophthalmic diseases according to the present invention is applied to the eyelids and the case where it is applied to the back, and the experimental results also indicate that the preparation according to the present invention is applied to the eyelids, whereby the drug is sustainedly transferred at a high concentration.

It is further apparent that the concentration of the drug in the conjunctivae of the other eye than the eye, to the eyelids of which the preparation has been applied is clearly lower than that in the eye patched, and that since the concentration of the drug in the plasma was lower than the detection limit value (< 0.005 μg/mL), the drug is percutaneously transferred to the conjunctivae from the eyelid parts, to which the preparation is applied, rather than the transfer to the conjunctivae through the systemic blood flow when the preparation is applied to the eyelid parts.

[Example 4]

An SIS-based pressure-sensitive adhesive obtained by blending 100 parts by weight of a rosin resin (product of Arakawa Chemical Industries, Ltd., trade name "Pinecrystal KE311") as a tackifier with 100 parts by weight of a styrene-isoprene-styrene block copolymer (SIS; product of Zeon Corporation, trade name "Quintac 3520") was used as a pressure-sensitive adhesive, and the SIS-based pressure-sensitive adhesive, ketotifen fumarate, KOH and isopropyl palmitate as percutaneous absorption enhancers (percutaneous permeation enhancers), and butylhydroxytoluene as an antioxidant were dissolved in toluene in accordance with the formulation shown in Table 5 to obtain a coating fluid having a solid content of 50% by weight. This coating fluid was coated on release paper so as to give a dry coat thickness of 40 μm. After drying, a support (polyester film having a thickness of 12 μm) was laminated to provide a patch preparation.

Table 5

| Component | | [wt.%] |
|---|---|---|
| Ketotifen fumarate | Drug | 10.0 |

(continued)

| Component | | [wt.%] |
|---|---|---|
| SIS-based pressure-sensitive adhesive | Pressure-sensitive adhesive | 76.7 |
| KOH | Percutaneous absorption enhancer | 2.5 |
| Isopropyl palmitate | Percutaneous absorption enhancer | 10.0 |
| Butylhydroxytoluene | Antioxidant | 0.8 |

The transdermal drug delivery system for treatment of ophthalmic diseases obtained above was cut to the size of 1 cm x 2 cm = 2 $cm^2$ and applied to a lower eyelid of one eye of each subject animal. The amounts of the drug (ketotifen fumarate) transferred to the conjunctiva and tear fluid were determined in accordance with the testing method for the transferability described in Example 3. The results are shown in Table 6.

Table 6

| Results of the test for transferability to the conjunctiva and tear fluid | | | | |
|---|---|---|---|---|
| Time | Application to lower eyelid part | | | |
| | Conjunctiva [$\mu$g/g·tissue] | | Tear fluid [$\mu$g/mL] | |
| | Conjunctiva of eye patched | Conjunctiva of the other eye | Tear fluid of eye patched | Tear fluid of the other eye |
| 4 | 0.046±0.013 | N.D. | 0.054±0.052 | N.D. |
| 8 | 0.078±0.046 | 0.011±0.020 | 0.193±0.139 | N.D. |
| *Mean ± S.D. (n = 3), conjunctiva: N.D. < 0.005 $\mu$g/g, tear fluid: N.D. < 0.05 $\mu$g/mL. | | | | |

As apparent from the comparative results between the amounts of the drug transferred to the conjunctiva and tear fluid of the eye under the application and the amounts of the drug transferred to the conjunctiva and tear fluid of the other eye, to which no preparation is applied, as shown in Table 6, it is understood that when the transdermal drug delivery system for treatment of ophthalmic diseases according to the present invention is applied to the skin surface of the eyelid, the drug is transferred to the conjunctiva and tear fluid sustainedly and at a high concentration. It is apparent that since the concentration of the drug in the plasma was lower than the detection limit value (< 0.005 $\mu$g/mL) after 4 hours elapsed in the other eye, the drug is percutaneously and sustainedly transferred to the conjunctiva and tear fluid of the eye, to which the preparation was applied, by applying the preparation according to the present invention to the skin surface of the eyelid.

Even from the experimental results shown in Table 6, it is apparent that the transfer of the drug to the anterior ocular segment (conjunctiva, lacrimal tissue, etc.) from the transdermal drug delivery system for treatment of ophthalmic diseases according to the present invention is conducted by percutaneous transfer from the applied site rather than the transfer through the systemic blood flow.

[Example 5]

The transdermal drug delivery system for treatment of ophthalmic diseases containing ketotifen fumarate obtained in Example 4 was used to make efficacy evaluation making use of a guinea pig histamine-induced chemosis model in accordance with the following test method. The results are shown in Table 7.

<Evaluation test by guinea pig histamine-induced chemosis model>

1. Testing method:

1) Animal

As animals, male Hartley guinea pigs aged 4 weeks were purchased from Japan SLC and respectively reared under conditions of a temperature of 23 ± 2°C and a humidity of 55 ± 10% in an SPF rearing room.

2) Group division and administration schedule (each group: n =. 5-6)

· Physiological saline group (control group)
· 2-hr Zaditen(R) instillation group
· 4-hr Zaditen(R) instillation group
· 8-hr Zaditen(R) instillation group
· 0.5-hr Example 4-preparation application group
· 4-hr Example 4-preparation application group
· 8-hr Example 4-preparation application group
· 10-hr (removed in 2 hr) Example 4-preparation application group

3) Preparation of histamine solution

A histamine solution was prepared by dissolving histamine dihydrochloride (Lot No. TCN1070, Wako Pure Chemical Industries, Ltd.) in physiological saline so as to give a concentration of 0.2% by weight. The histamine solution thus prepared was filtered through a filter [MILLEX(R)-GV] having a pore size of 0.22 $\mu$m to remove impurities.

4) Preparation of dye (Evans blue) solution

A dye solution was prepared by dissolving Evans blue (Lot No. K25612469, Merk) in physiological saline so as to give a concentration of 2% by weight. The dye solution thus prepared was filtered through a filter [MILLEX(R)-GV] having a pore size of 0.22 $\mu$m to remove impurities.

5) Induction of chemosis by histamine and administration of test substance

An equi-amount mixture of a 50 mg/mL ketamine injection [Ketalal(R) 50 for animals, product of Sankyo Pharmaceutical Co., Ltd.] and a 20 mg/mL xylazine injection [Celactal(R) 2% injection, product of Bayer Ltd.] was administered (1-mL syringe, 25-G needle) in an amount of 0.5 mL/body in the femoral muscle of a hind limb of the guinea pig to anesthetize it. Under the anesthesia, 1 mL/kg (20 mg/kg) of the 2 wt.% Evans blue solution was injected (1-mL syringe, 30-G needle) from an ear vein of the guinea pig. Just after the injection, 50 $\mu$L of the 0.2 wt.% aqueous histamine solution was injected (glass syringe equipped with a 1/5 hypodermic needle) in the conjunctivae of the lower eyelids of both eyes in order from left to right to induce conjunctivitis. After 30 minutes from the induction of conjunctivitis, the guinea pig was euthanized by a guillotine method. The hair of the head was then clipped with an electric hair clipper to enucleate the eyelid and conjunctiva sites dyed blue by sthenia of vascular permeability attending on blepharoconjunctivitis.
The administration of the test substances will be described below.

· Physiological saline: administered in an amount of 10 $\mu$L 0.5 hours prior to the induction of conjunctivitis.
· Zaditen ophthalmic solution: administered by instillation in an amount of 10 $\mu$L 0.5, 4 and 8 hours prior to the induction of conjunctivitis.
· Patch preparation of Example 4: applied in an area of 0.5 $cm^2$ (0.5 cm x 1 cm) to the skin (the hair of which had been removed) of an lower eyelid of a left eye of the guinea pig 0.5, 4 and 8 hours and 10 hours (removed in 2 hours) prior to the induction of conjunctivitis.

6) Enucleation of chemosis site and determination of amount of dye leaked

After enucleation of the chemosis site, it was immersed in 0.8 mL of a 1N (mol/L) potassium hydroxide solution and incubated ($CO_2$ Incubator MCO-345, SANYO) overnight at 37°C to dissolve the conjunctiva tissue. To this solution, was added 7.2 mL of a 5:13 (V:V) mixture of 0.6N phosphoric acid and acetone, and the resultant mixture was stirred, thereby conducting neutralization and dye extraction. After the extract was centrifuged (3,000 rpm, 15 min), an absorbance at 620 nm of supernatant was measured by means of a spectrophotometer [GLP Instrument No. 93: U-3000, Hitachi Ltd.]. At the same time, an absorbance of the Evans blue standard solution was measured, and the amount of the dye leaked in each sample was converted on the basis of this value.

7) Evaluation method

The inhibition effect on chemosis was evaluated by the amount of the dye leaked in each group and an inhibition rate calculated out by the following equation.

$$\texttt{Inhibition rate (\%) = \{1 - (X/N)\} x 100}$$

X: average value of the amounts of the dye leaked in each group
N: average value of the amounts of the dye leaked in the physiological saline (control) group

Table 7

| Evaluation results in guinea pig histamine-induced chemosis model (results as to the eye patched) | | |
|---|---|---|
| Group | Inhibition rate [%] | Number of cases |
| Zaditen instillation 0.5 hr | 80.7 $\pm$ 1.6 | 5 |
| Zaditen instillation 4 hr | 46.3 $\pm$ 4.8 | 6 |
| Zaditen instillation 8 hr | 32.8 $\pm$ 15.0 | 6 |
| Example 4-preparation application 0.5 hr | 42.4 $\pm$ 3.2 | 6 |
| Example 4-preparation application 4 hr | 79.5 $\pm$ 3.5 | 5 |
| Example 4-preparation application 8 hr | 83.4 $\pm$ 1.0 | 6 |
| Example 4-preparation application 2 hr (8 hr after removal) | 75.4 $\pm$ 1.9 | 6 |
| mean $\pm$ standard error<br>Inhibition rate = inhibition rate to the amount of the dye leaked in the conjunctiva attending on blepharoconjunctivitis and physiological saline group | | |

As apparent from the results shown in Table 7, it is understood that the transdermal drug delivery system for treatment of ophthalmic diseases according to the present invention sustains its efficacy over a long period of time compared with the instillation of the ophthalmic solution. Further, the transdermal drug delivery system for treatment of ophthalmic diseases according to the present invention sustains its efficacy even when it was removed 2 hours after applied to the skin surface of the eyelid, and 8 hours elapsed after the removal. It is thus apparent that the efficacy is sustained for a long period of time even after the preparation is removed.

INDUSTRIAL APPLICABILITY

According to the present invention, there is a novel preparation for treatment of ophthalmic diseases, by which a remedy for ophthalmic diseases can be sustainedly administered in an amount effective for treatment to an ophthalmic topical tissue and its efficacy can be sustainedly developed without causing such side effects as observed in a systemic preparation administered through a systemic blood flow.
According to the present invention, there is also provided use of the transdermal drug delivery system for treatment of ophthalmic diseases. According to the present invention, there is further provided a method for percutaneously transferring a remedy for ophthalmic diseases to an ophthalmic topical tissue using the transdermal drug delivery system for treatment of ophthalmic diseases.

**Claims**

1.  A transdermal drug delivery system for use in the treatment of ophthalmic diseases having a structure that is a plaster layer containing a remedy for ophthalmic diseases and a pressure-sensitive adhesive provided on a support, and
    wherein the system is applied to a front skin surface of an eyelid to transfer the remedy for ophthalmic diseases in the plaster layer to an external ophthalmictissue including a conjunctiva, lacrimal tissue and cornea by percutaneous permeation substantially without being administered through a systemic blood flow, and
    wherein the pressure-sensitive adhesive is a rubber-based pressure-sensitive adhesive, acrylic pressure-sensitive adhesive or silicone-based pressure-sensitive adhesive.

**2.** The transdermal drug delivery system for use in the treatment of ophthalmic diseases according to claim 1, wherein the remedy for ophthalmic diseases is an antiviral agent, antibacterial agent, anti-mycotic agent, antiallergic agent, anti-inflammatory agent, nonsteroidal anti-inflammatory agent, anti-inflammatory-analgesic agent, anti-inflammatory enzymatic agent, antibiotic, sulfa agent, synthetic penicillin, remedy for glaucoma, remedy for cataract, miotic, mydriatic, topical astringent, vasopressor, preventive for rise in ocular tension, remedy for ocular hypertension, surface anesthetic, $\alpha$1-blocker, $\beta$-blocker, $\beta$1-blocker, carbonic anhydrase inhibitor, topical selective H1-blocker, adrenal cortical hormone, vitamin B12, coenzyme type vitamin B2, anticholinesterase agent or organic iodine preparation.

**3.** The transdermal drug delivery system for use in the treatment of ophthalmic diseases according to claim 2, wherein the remedy for ophthalmic diseases is an antibacterial agent, antiallergic agent or nonsteroidal anti-inflammatory agent.

**4.** The transdermal drug delivery system for use in the treatment of ophthalmic diseases according to claim 2, wherein the remedy for ophthalmic diseases is a compound having a molecular weight of at most 1,000.

**5.** The transdermal drug delivery system for use in the treatment of ophthalmic diseases according to claim 1, wherein the remedy for ophthalmic diseases is an antibacterial agent, antiallergic agent or nonsteroidal anti-inflammatory agent having a molecular weight of at most 1,000.

**6.** The transdermal drug delivery system for use in the treatment of ophthalmic diseases according to claim 5, wherein the remedy for ophthalmic diseases is ketotifen fumarate or diclofenac sodium.

**7.** The transdermal drug delivery system for use in the treatment of ophthalmic diseases according to claim 1, wherein the rubber-based pressure-sensitive adhesive comprises a styrene-isoprene-styrene block copolymer as a pressure-sensitive adhesive base.

**8.** The transdermal drug delivery system for use in the treatment of ophthalmic diseases according to claim 1, wherein the acrylic pressure-sensitive adhesive is a (co)polymer of at least one alkyl (meth)acrylate, or a copolymer of an alkyl (meth)acrylate and a functional monomer or vinyl ester monomer copolymerizable with this ester or both monomers.

**9.** The transdermal drug delivery system for use in the treatment of ophthalmic diseases according to claim 1, wherein the plaster layer contains a percutaneous absorption enhancer.

**10.** The transdermal drug delivery system for use in the treatment of ophthalmic diseases according to claim 9, wherein the percutaneous absorption enhancer is an aliphatic alcohol, fatty acid, fatty acid ester, alcohol amine, polyhydric alcohol alkyl ether, polyoxyethylene alkyl ether, glyceride, middle-chain fatty acid ester of a polyhydric alcohol, lactic acid alkyl ester, dibasic acid alkyl ester, acylated amino acid, pyrrolidone or its derivative, lactic acid, tartaric acid, 1,2,6-hexanetriol, benzyl alcohol, lanoline, potassium hydroxide (KOH), tris(hydroxymethyl)aminomethane, or a mixture of 2 or more compounds thereof.

**11.** The transdermal drug delivery system for use in the treatment of ophthalmic diseases according to claim 9, wherein the percutaneous absorption enhancer is an aliphatic higher alcohol, fatty acid, alcohol amine, fatty acid ester, polyoxyethylene alkyl ether, KOH, tris(hydroxymethyl)aminomethane, or a mixture of two or more compounds thereof.

**12.** The transdermal drug delivery system for use in the treatment of ophthalmic diseases according to claim 9, wherein the pressure-sensitive adhesive layer is a rubber-based pressure-sensitive adhesive containing 100 parts by weight of the styrene-isoprene-styrene block copolymer, 10 to 400 parts by weight of a tackifier, 1 to 50 parts by weight of the percutaneous absorption enhancer and 0.1 to 60 parts by weight of the remedy for ophthalmic diseases.

**13.** The transdermal drug delivery system for use in the treatment of ophthalmic diseases according to claim 9, wherein the pressure-sensitive adhesive is an acrylic pressure-sensitive adhesive containing 100 parts by weight of the acrylic (co)polymer, 1 to 50 parts by weight of the percutaneous absorption enhancer and 0.1 to 60 parts by weight of the remedy for ophthalmic diseases.

**14.** The transdermal drug delivery system for use in the treatment of ophthalmic diseases according to claim 1, which has a form capable of being applied along a front skin surface of an upper eyelid, a lower eyelid or both eyelids.

15. The transdermal drug delivery system for use in the treatment of ophthalmic diseases according to claim 1, wherein when the system is applied to the front skin surface of the eyelid, the amount (unit: μg/g tissue) of the remedy transferred to an external ophthalmic tissue under the application within 8 hours after the application amounts to at least twice as much as the amount of the remedy transferred to the external ophthalmic tissue through a systemic blood flow.

**Patentansprüche**

1. Ein transdermales Arzneimittelzuführungssystem zur Verwendung bei der Behandlung von Augenerkrankungen, das eine Struktur aufweist, das eine Pflasterschicht, die ein Heilmittel gegen Augenerkrankungen enthält, und ein druckempfindlicher Klebstoff, der auf einem Träger bereitgestellt wird, ist,
wobei das System auf eine vordere Hautoberfläche eines Augenlids appliziert wird, um das Heilmittel gegen Augenerkrankungen in der Pflasterschicht auf ein äußeres topisches Augengewebe, einschließlich einer Augenbindehaut, Tränenganggewebe und Hornhaut, durch perkutane Permeation zu übertragen, im Wesentlichen ohne durch einen systemischen Blutkreislauf verabreicht zu werden, und
wobei der druckempfindliche Klebstoff ein druckempfindlicher Klebstoff auf Gummigrundlage, druckempfindlicher Acrylklebstoff oder druckempfindlicher Klebstoff auf Silikongrundlage ist.

2. Das transdermale Arzneimittelzuführungssystem zur Verwendung bei der Behandlung von Augenerkrankungen gemäß Anspruch 1, wobei das Heilmittel gegen Augenerkrankungen ein antivirales Mittel, antibakterielles Mittel, Antipilzmittel, Antiallergikum, Entzündungshemmer, nichtsteroidaler Entzündungshemmer, analgetischer Entzündungshemmer, enzymatischer Entzündungshemmer, Antibiotikum, Sulfonamidmittel, synthetisches Penicillin, Heilmittel gegen Glaukom, Heilmittel gegen Katarakt, Miotikum, Mydriatikum, topisches Adstringens, blutdrucksteigerndes Mittel, präventives Mittel gegen einen Anstieg des Augendrucks, Heilmittel gegen Augenhochdruck, Oberflächenanästhetikum, α1-Blocker, β-Blocker, β1-Blocker, Carboanhydrasehemmer, topischer selektiver H1-Blocker, Nebennierenrindenhormon, Vitamin B12, Coenzym vom Typ Vitamin B2, Anticholinesterasemittel oder organisches Iodpräparat ist.

3. Das transdermale Arzneimittelzuführungssystem zur Verwendung bei der Behandlung von Augenerkrankungen gemäß Anspruch 2, wobei das Heilmittel gegen Augenerkrankungen ein antibakterielles Mittel, Antiallergikum oder nichtsteroidaler Entzündungshemmer ist.

4. Das transdermale Arzneimittelzuführungssystem zur Verwendung bei der Behandlung von Augenerkrankungen gemäß Anspruch 2, wobei das Heilmittel gegen Augenerkrankungen eine Verbindung mit einem Molekulargewicht von höchstens 1000 ist.

5. Das transdermale Arzneimittelzuführungssystem zur Verwendung bei der Behandlung von Augenerkrankungen gemäß Anspruch 1, wobei das Heilmittel gegen Augenerkrankungen ein antibakterielles Mittel, Antiallergikum oder nichtsteroidaler Entzündungshemmer mit einem Molekulargewicht von höchstens 1000 ist.

6. Das transdermale Arzneimittelzuführungssystem zur Verwendung bei der Behandlung von Augenerkrankungen gemäß Anspruch 5, wobei das Heilmittel gegen Augenerkrankungen Ketotifenfumarat oder Diclofenac-Natrium ist.

7. Das transdermale Arzneimittelzuführungssystem zur Verwendung bei der Behandlung von Augenerkrankungen gemäß Anspruch 1, wobei der druckempfindliche Klebstoff auf Gummigrundlage ein Styrol-Isopren-Styrol-Blockcopolymer als druckempfindliche Klebstoffgrundlage beinhaltet.

8. Das transdermale Arzneimittelzuführungssystem zur Verwendung bei der Behandlung von Augenerkrankungen gemäß Anspruch 1, wobei der druckempfindliche Acrylklebstoff ein (Co-)Polymer von mindestens einem Alkyl(meth)acrylat ist oder ein Copolymer von einem Alkyl(meth)acrylat und einem funktionalen Monomer oder Vinylestermonomer ist, das mit diesem Ester oder beiden Monomeren copolymerisierbar ist.

9. Das transdermale Arzneimittelzuführungssystem zur Verwendung bei der Behandlung von Augenerkrankungen gemäß Anspruch 1, wobei die Pflasterschicht einen perkutanen Absorptionsverstärker enthält.

**10.** Das transdermale Arzneimittelzuführungssystem zur Verwendung bei der Behandlung von Augenerkrankungen gemäß Anspruch 9, wobei der perkutane Absorptionsverstärker ein aliphatischer Alkohol, eine Fettsäure, ein Fettsäureester, Alkoholamin, mehrwertiger Alkoholalkylether, Polyoxyethylenalkylether, Glycerid, mittelkettiger Fettsäureester eines mehrwertigen Alkohols, Milchsäurealkylester, zweibasiger Säurenalkylester, eine acylierte Aminosäure, ein Pyrrolidon oder sein Derivat, eine Milchsäure, Weinsäure, ein 1,2,6-Hexantriol, Benzylalkohol, Lanolin, Kaliumhydroxid (KOH), Tris(hydroxymethyl)aminomethan oder ein Gemisch von 2 oder mehr Verbindungen davon ist.

**11.** Das transdermale Arzneimittelzuführungssystem zur Verwendung bei der Behandlung von Augenerkrankungen gemäß Anspruch 9, wobei der perkutane Absorptionsverstärker ein aliphatischer höherer Alkohol, eine Fettsäure, ein Alkoholamin, Fettsäureester, Polyoxyethylenalkylether, KOH, Tris(hydroxymethyl)aminomethan oder ein Gemisch von 2 oder mehr Verbindungen davon ist.

**12.** Das transdermale Arzneimittelzuführungssystem zur Verwendung bei der Behandlung von Augenerkrankungen gemäß Anspruch 9, wobei die druckempfindliche Klebstoffschicht eine druckempfindliche Klebstoffschicht auf Gummigrundlage ist, die 100 Gewichtsteile des Styrol-Isopren-Styrol-Blockcopolymers, 10 bis 400 Gewichtsteile eines Klebrigmachers, 1 bis 50 Gewichtsteile des perkutanen Absorptionsverstärkers und 0,1 bis 60 Gewichtsteile des Heilmittels gegen Augenerkrankungen enthält.

**13.** Das transdermale Arzneimittelzuführungssystem zur Verwendung bei der Behandlung von Augenerkrankungen gemäß Anspruch 9, wobei die druckempfindliche Klebstoffschicht eine druckempfindliche Acrylklebstoffschicht ist, die 100 Gewichtsteile des Acryl(co)polymers, 1 bis 50 Gewichtsteile des perkutanen Absorptionsverstärkers und 0,1 bis 60 Gewichtsteile des Heilmittels gegen Augenerkrankungen enthält.

**14.** Das transdermale Arzneimittelzuführungssystem zur Verwendung bei der Behandlung von Augenerkrankungen gemäß Anspruch 1, das eine Form aufweist, die entlang einer vorderen Hautoberfläche eines oberen Augenlids, eines unteren Augenlids oder beider Augenlider appliziert werden kann.

**15.** Das transdermale Arzneimittelzuführungssystem zur Verwendung bei der Behandlung von Augenerkrankungen gemäß Anspruch 1, wobei, wenn das System auf die vordere Hautoberfläche des Augenlids appliziert wird, die Heilmittel (Einheit: μg/g·Gewebe), die im Rahmen der Applikation auf ein äußeres Augengewebe innerhalb von 8 Stunden nach der Applikation übertragen wird, mindestens das Zweifache der durch einen systemischen Blutkreislauf auf das äußere Augengewebe übertragenen Heilmittelmenge beträgt.

**Revendications**

**1.** Système d'administration d'un médicament transdermique à utiliser dans le traitement de maladies ophtalmiques ayant une structure constituée d'une couche pansement contenant un remède pour maladies ophtalmiques et un adhésif sensible à la pression fourni sur un support, et
dans lequel le système est appliqué sur une surface cutanée frontale d'une paupière en vue de transférer le remède pour maladies ophtalmiques contenu dans la couche pansement à un tissu ophtalmique externe incluant une conjonctive, le tissu lacrymal et la cornée par pénétration percutanée sans être administré par le biais d'un débit sanguin systémique, et
dans lequel l'adhésif sensible à la pression est un adhésif sensible à la pression à base de caoutchouc, un adhésif acrylique sensible à la pression ou un adhésif sensible à la pression à base de silicone.

**2.** Système d'administration d'un médicament transdermique à utiliser dans le traitement de maladies ophtalmiques selon la revendication 1, dans lequel le remède pour maladies ophtalmiques est un agent antiviral, un agent antibactérien, un agent anti-mycotique, un agent anti-allergique, un agent anti-inflammatoire, un agent anti-inflammatoire non stéroïdien, un agent analgésique anti-inflammatoire, un agent enzymatique anti-inflammatoire, un antibiotique, un agent sulfamidé, une pénicilline synthétique, un remède pour le glaucome, un remède pour la cataracte, miotique, mydriatique, astringent topique, vasopresseur, préventif de la poussée de la tension oculaire, un remède pour l'hypertension oculaire, un anesthésique surfactant, un agent α1-bloquant, un agent β-bloquant, un agent β-bloquant, un inhibiteur de l'anhydrase carbonique, un agent H1-bloquant sélectif topique, une hormone corticosurrénale, la vitamine B12, la vitamine B2 de type coenzyme, un agent anticholinestérase ou une préparation à base d'iode organique.

**3.** Système d'administration d'un médicament transdermique à utiliser dans le traitement de maladies ophtalmiques selon la revendication 2, dans lequel le remède pour maladies ophtalmiques est un agent antibactérien, un agent anti-allergique ou un agent anti-inflammatoire non stéroïdien.

4. Système d'administration d'un médicament transdermique à utiliser dans le traitement de maladies ophtalmiques selon la revendication 2, dans lequel le remède pour maladies ophtalmiques est un composé ayant un poids moléculaire de 1000 au maximum.

5. Système d'administration d'un médicament transdermique à utiliser dans le traieemnt de maladies ophtalmiques selon la revendication 1, dans lequel le remède pour maladies ophtalmiques est un agent antibactérien, un agent anti-allergique ou un agent anti-inflammatoire non stéroïdien ayant un poids moléculaire de 1000 au maximum.

6. Système d'administration d'un médicament transdermique à utiliser dans le traitement de maladies ophtalmiques selon la revendication 5, dans lequel le remède pour maladies ophtalmiques est le fumarate de kétotifène ou le sodium de diclofénac.

7. Système d'administration d'un médicament transdermique à utiliser dans le traitement de maladies ophtalmiques selon la revendication 1, dans lequel l'adhésif sensible à la pression à base de caoutchouc comprend un copolymère séquencé de styrène-isoprène-styrène en tant que base adhésive sensible à la pression.

8. Système d'administration d'un médicament transdermique à utiliser dans le traitement de maladies ophtalmiques selon la revendication 1, dans lequel l'adhésif acrylique sensible à la pression est un (co)polymère d'au moins un alkyl-(méth)acrylate, ou un copolymère d'un alkyl(méth)acrylate et un monomère fonctionnel ou un monomère d'ester de vinyle copolymérisable avec ledit ester ou avec les deux monomères.

9. Système d'administration d'un médicament transdermique à utiliser dans le traitement de maladies ophtalmiques selon la revendication 1, dans lequel la couche pansement contient un activateur de l'absorption percutanée.

10. Système d'administration d'un médicament transdermique à utiliser dans le traitement de maladies ophtalmiques selon la revendication 9, dans lequel l'activateur de l'absorption percutanée est un alcool aliphatique, un acide gras, un ester d'acide gras, un alcool aminé, un éther d'alkyle d'alcool polyhydrique, un éther d'alkyle de polyoxyéthylène, un glycéride, un ester d'acide gras à chaîne moyenne d'un alcool polyhydrique, un ester d'alkyle d'acide lactique, un ester d'alkyle d'acide dibasique, un acide aminé acylé, la pyrrolidone ou son dérivé, l'acide lactique, l'acide tartarique, le 1,2,6-hexanétriol, l'alcool benzylique, la lanoline, l'hydroxyde de potassium (KOH), le tris(hydroxyméthyl)aminométhane, ou un mélange de 2 ou de plusieurs composés de ces derniers.

11. Système d'administration d'un médicament transdermique à utiliser dans le traitement de maladies ophtalmiques selon la revendication 9, dans lequel l'activateur de l'absorption percutanée est un alcool aliphatique supérieur, un acide gras, un alcool aminé, un ester d'acide gras, un éther d'alkyle de polyoxyéthylène, le KOH, le tris(hydroxyméthyl)aminométhane, ou un mélange de deux ou de plusieurs composés de ces derniers.

12. Système d'administration d'un médicament transdermique à utiliser dans le traitement de maladies ophtalmiques selon la revendication 9, dans lequel la couche d'adhésif sensible à la pression est un adhésif sensible à la pression à base de caoutchouc contenant 100 parties en poids du copolymère séquencé styrène-isoprène-styrène, 10 à 400 parties en poids d'un agent donnant du collant, 1 à 50 parties en poids de l'activateur de l'absorption percutanée et 0,1 à 60 parties en poids du remède pour maladies ophtalmiques.

13. Système d'administration d'un médicament transdermique à utiliser dans le traitement de maladies ophtalmiques selon la revendication 9, dans lequel l'adhésif sensible à la pression est un adhésif acrylique sensible à la pression contenant 100 parties en poids du (co)polymère acrylique, 1 à 50 parties en poids de l'activateur de l'absorption percutanée et 0,1 à 60 parties en poids du remède pour maladies ophtalmiques.

14. Système d'administration d'un médicament transdermique à utiliser dans le traitement de maladies ophtalmiques selon la revendication 1, qui a une forme capable d'être appliquée le long d'une surface cutanée frontale d'une paupière supérieure, d'une paupière inférieure ou des deux paupières.

15. Système d'administration d'un médicament transdermique à utiliser dans le traitement de maladies ophtalmiques selon la revendication 1 dans lequel, quand le système est appliqué à la surface cutanée frontale de la paupière, la quantité (unité : $\mu$g/g.tissu) du remède transféré à un tissu ophtalmique externe sous l'application dans les 8 heures suivant l'application équivaut à au moins deux fois la quantité du remède transféré au tissu ophtalmique externe par le biais d'un débit sanguin systémique.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000256214 A
- JP 8509716 PCT
- WO 0126648 A
- US 6277855 B